(19) Europäisches Patentamt
European Patent Office
Office européen des brevets

(11) Veröffentlichungsnummer: **0 346 658 B1**

# (12) EUROPÄISCHE PATENTSCHRIFT

(45) Veröffentlichungstag der Patentschrift: **01.09.93**

(51) Int. Cl.5: **C07F 9/547**, C07F 9/53, C07F 9/40, C07F 9/32, C07K 5/08

(21) Anmeldenummer: **89109313.0**

(22) Anmeldetag: **24.05.89**

(54) **Verfahren zur Herstellung phosphorhaltiger L-Aminosäuren sowie ihrer Ester und N-Derivate.**

(30) Priorität: **27.05.88 DE 3817956**

(43) Veröffentlichungstag der Anmeldung:
**20.12.89 Patentblatt 89/51**

(45) Bekanntmachung des Hinweises auf die Patenterteilung:
**01.09.93 Patentblatt 93/35**

(84) Benannte Vertragsstaaten:
**BE CH DE FR GB IT LI NL**

(56) Entgegenhaltungen:
**EP-A- 0 224 880**

(73) Patentinhaber: **HOECHST AKTIENGESELL-SCHAFT**

**D-65926 Frankfurt(DE)**

(72) Erfinder: **Zeiss, Hans-Joachim, Dr.**
**Hauptstrasse 127**
**D-6231 Sulzbach(DE)**

EP 0 346 658 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein Verfahren zur Herstellung von phosphorhaltigen L-Aminosäuren der Formel I oder deren Salzen

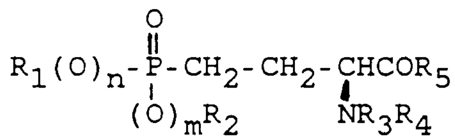

(I),

worin

$R_1$ und $R_2$ unabhängig voneinander Wasserstoff, $(C_1\text{-}C_6)$-Alkyl, das unsubstituiert ist oder durch Halogen oder $(C_6\text{-}C_{10})$-Aryl ein- oder mehrfach substituiert ist, oder $(C_3\text{-}C_{10})$-Cycloalkyl,

$R_3$ Wasserstoff, $(C_1\text{-}C_6)$-Alkyl, das unsubstituiert ist oder ein- oder mehrfach durch Halogen und/oder einfach durch Hydroxy substituiert ist, $(C_1\text{-}C_{10})$-Acyl, das ein- oder mehrfach durch Halogen oder $(C_1\text{-}C_6)$-Alkyl oder einfach durch $(C_6\text{-}C_{10})$-Aryl, $(C_1\text{-}C_6)$-Alkoxy oder $(C_1\text{-}C_6)$-Alkoxy, das durch $(C_6\text{-}C_{13})$-Aryl substituiert ist, substituiert sein kann,

$R_4$ Wasserstoff oder 1-Hydroxy-$(C_1\text{-}C_6)$-alkyl, das unsubstituiert oder ein- oder mehrfach durch Halogen substituiert ist,

$R_5$ Hydroxy, $(C_1\text{-}C_6)$-Alkoxy, Amino, Ala-Ala-$OR_6$ oder Ala-Leu-$OR_6$,

$R_6$ Wasserstoff, $(C_1\text{-}C_6)$-Alkyl oder Benzyl,

n die Zahl null oder eins und

m die Zahl null, wenn $R_2$ nicht Wasserstoff ist, oder 1

bedeuten, dadurch gekennzeichnet, daß man

a) eine Verbindung der Formel II

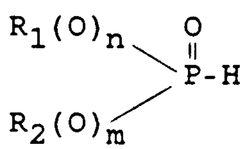

(II),

wobei $R_1$, $R_2$ die obengenannte Bedeutung besitzt mit der Ausnahme, daß $R_1$ nicht Wasserstoff sein darf, wenn n = 1 ist, mit einer Verbindung der Formel III

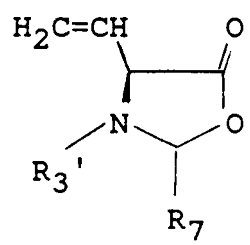

(III),

worin

$R_3'$ die Bedeutung von $R_3$ außer Wasserstoff besitzt und

$R_7$ H, $(C_1\text{-}C_5)$-Alkyl, das unsubstituiert ist oder ein-oder mehrfach durch Halogen substituiert ist,

bedeutet, in Gegenwart katalytischer Mengen eines Radikalbildners umsetzt und

b) das erhaltene Zwischenprodukt der Formel IV

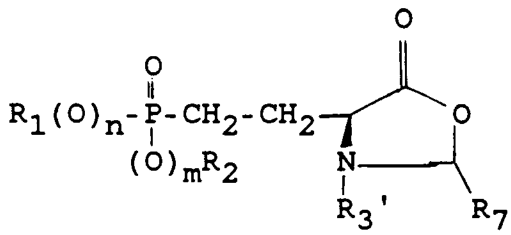

(IV)

mit HCl, einem Alkalihydroxid, Alkalialkoholat, $NH_3$, H-Ala-Ala-$OR_6$ oder H-Ala-Leu-$OR_6$, wobei $R_6$ die obengenannte Bedeutung außer Wasserstoff besitzt, umsetzt und die erhaltene Verbindung der Formel I gegebenenfalls auf üblichem Wege derivatisiert.

Unter ($C_6$-$C_{10}$)-Aryl ist bei den obigen Definitionen insbesondere Phenyl oder Naphthyl zu verstehen. ($C_6$-$C_{13}$)-Aryl bedeutet insbesondere Phenyl, Naphthyl oder Fluorenyl. Beispiele für die bei $R_3$ genannten substituierten Acylreste sind Dichlor-, Trichlor- oder Trifluoracetyl, Benzyloxycarbonyl, Fluorenylmethoxycarbonyl, Naphthylmethoxycarbonyl.

Die Verbindungen der Formel I sind z. B. aus Bull. Chem. Soc. Japan 60, 1761 (1987), DE-OS 2 856 260 und Sci. Rep. Meiji Seika Kaisha 13, 34 (1973) bekannt. Dort werden auch deren herbizide, fungizide und antivirale Eigenschaften beschrieben. Letztere Eigenschaft gilt speziell für L-2-Amino-4-phosphonobuttersäure. Ferner ist L-3-Amino-3-carboxypropan-phosphonige Säure ein wichtiges Zwischenprodukt bei der fermentativen Herstellung der betreffenden Herbizide. (J. Antibiotics 36, 96 (1983).

Hervorzuheben unter den Verbindungen der Formel I sind die Verbindungen, worin $R_1(O)_n$ = $CH_3$ oder $C_2H_5$, $R_2(O)_m$ = OH, $R_3$ und $R_4$ = H und $R_5$ = OH, Ala-Ala-OH oder Ala-Leu-OH bedeuten und deren Salze. Besondere Bedeutung besitzt das Herbizid Glufosinate (4-[Hydroxy(methyl)phosphinoyl]homoalanin, Pesticide Manual, 8. Aufl. (1987), S. 448).

Gemäß DE-OS 28 56 260 ist die herbizide Wirkung des L-Isomeren von Glufosinate doppelt so groß wie die des Racemats. Daher bietet der Einsatz des L-Isomeren eindeutige Vorteile.

Das erfindungsgemäße Verfahren liefert die L-Isomeren der Formel I in hohen optischen Ausbeuten von über 90 %, was einem Gehalt von 95 % des L-Isomeren entspricht.

Phosphorhaltige L-Aminosäuren können mit hoher optischer Reinheit bisher nur durch aufwendige, enzymatische Racematspaltungsverfahren erhalten werden (DE-OS 29 39 269 und DE-OS 30 48 612).

Daneben sind zwar auch Verfahren bekannt, die sich einer enantioselektiven Synthese bedienen, jedoch besitzen diese verschiedene Nachteile.

So ergibt z. B. die in EP-OS 127 429 beschriebene enantioselektive Alkylierung von chiralen Schiff-Basen nur optische Ausbeuten von maximal 78 %, während die in DE-OS 36 09 818 beschriebene asymmetrische Hydrierung von 2,3-Dehydroaminosäuren von schwer zugänglichen Ausgangsmaterialien ausgeht.

Daneben sind zwei Verfahren bekannt, die von heterocyclischen Vorstufen ausgehen (DE-OS 35 42 645 und DE-OS 35 25 267).

Diese Verfahren haben jedoch den Nachteil, daß die betreffenden heterocyclischen Ausgangsverbindungen nur in mehrstufigen aufwendigen Syntheseschritten herstellbar sind, während die Ausgangsverbindungen der Formel III des erfindungsgemäßen Verfahrens aus der gut zugänglichen L-Glutaminsäure auch in einem technischen Produktionsverfahren einfach herzustellen sind.

Der Verfahrensschritt a) des erfindungsgemäßen Verfahrens wird im Temperaturbereich zwischen 50° und 200°C, bevorzugt zwischen 70° und 140°C durchgeführt.

Als Radikalbildner kommen alle die bekannten Verbindungen in Frage, die im Temperaturbereich von 50° - 200°C Radikale bilden.

Beispiele für solche Verbindungen sind:
Organische Peroxide wie Di-tert.-Butylperoxid oder Dibenzoylperoxid, Carbonsäureperester wie tert.-Butylperpivalat, tert.-Butylperoctoat, tert.-Amylperpivalat, tert.-Butylperneodecanoat oder tert.-Amylperneodecanoat oder aliphatische Azoverbindungen wie Azobisisobutyronitril.

Anstelle chemischer Radikalbildner können auch energiereiche, zur Radikalbildung führende Strahlen wie UV-, $\gamma$- oder Röntgenstrahlen verwendet werden. Ebenso kann der Katalysator aus Gemischen von zweien oder mehreren der aufgeführten Radikalbildner bestehen.

Der Verfahrensschritt a) wird im allgemeinen so durchgeführt, daß man die ungesättigte Verbindung der allgemeinen Formel III mit der 1- bis 10-fachen Menge, vorzugsweise mit dem 1,5- bis 4-fachen Überschuß

der Phosphorwasserstoffverbindung der allgemeinen Formel II ohne Lösungsmittel oder in Gegenwart eines hochsiedenden, inerten Lösungsmittels wie Toluol oder Xylol auf die Reaktionstemperatur erwärmt und mit 0,1 bis 20 Molprozent, vorzugsweise jedoch mit 0,5 bis 10 Molprozent des radikalbildenden Katalysators, bezogen auf die Komponente der allgemeinen Formel III, versetzt.

Eine andere Verfahrensweise besteht darin, daß man die Phosphorwasserstoffverbindung der allgemeinen Formel II vorlegt, auf Reaktionstemperatur erwärmt und mit einer Mischung der ungesättigten Verbindung III und des Katalysators, gegebenenfalls gelöst in einem hochsiedenden, inerten Lösungsmittel, versetzt.

Zur Vermeidung unerwünschter Nebenreaktionen ist es ratsam, die Reaktion in einer Schutzgasatmosphäre durchzuführen. Als Schutzgase kommen Stickstoff, Argon oder Kohlendioxid in Frage.

Nach beendeter Reaktion wird die im Überschuß vorhandene Phosphorwasserstoffkomponente II, gegebenenfalls zusammen mit dem Lösungsmittel, durch bekannte Verfahren wie z. B. Destillation vom Reaktionsprodukt abgetrennt. Das rohe Reaktionsprodukt kann durch bekannte Verfahren wie z. B. Chromatographie weiter gereinigt werden.

Überraschenderweise findet bei den beschriebenen Reaktionsbedingungen keine radikalische Polymerisation der substituierten Vinylverbindung III, sondern eine glatte Reaktion zum Zwischenprodukt der Formel IV statt, vgl. G. Henrici-Olive, S. Olive: Polymerisation, S. 1 ff, Verlag Chemie, Weinheim (1969).

Außerdem war nicht zu erwarten, daß eine regioselektive Addition der Komponente II an die Doppelbindung von III eintritt, da bei ähnlichen Reaktionen die Bildung von Isomerengemischen beobachtet wurde, s. Tetrahedron Letters 1984, 4737.

Weiterhin ist bemerkenswert, daß trotz der relativ hohen Reaktionstemperaturen keine Racemisierung eintritt.

Die Verbindungen der Formel III sind aus Tetrahedron Letters 25, 1425 (1984) bekannt oder können nach den dort beschriebenen Methoden hergestellt werden. Ihre Herstellung erfolgt aus der gut zugänglichen L-Glutaminsäure.

Die Verbindungen der Formel II werden in Houben-Weyl, Methoden der organischen Chemie, Band XII/1, S. 1, 5, 193 und 294 (1963), Georg-Thieme-Verlag, Stuttgart, beschrieben.

Bei Verfahrensschritt b) werden bei Einsatz von HCl die Aminosäuren ($R_5$ = OH) in Form ihrer Hydrochloride erhalten.

Insbesondere wird wäßrige 3N - 12N HCl, besonders bevorzugt 5N - 10N HCl verwendet.

Zweckmäßigerweise wird die Verbindung der Formel IV in der wäßrigen Salzsäure gelöst und mehrere Stunden auf 90° bis 130°C erhitzt. Die saure, wäßrige Lösung wird dann mit einem nicht mit Wasser mischbaren Lösungsmittel wie Toluol, Xylol, Dichlormethan oder Methylisobutylketon extrahiert, um die Folgeprodukte der Abspaltung der Reste $R_3$, $R_4$ oder gegebenenfalls $R_1$, $R_2$ zu entfernen. Die wäßrige Lösung wird vollständig eingeengt und das Rohprodukt gegebenenfalls durch bekannte Maßnahmen wie Umkristallisation oder Ionenaustausch gereinigt. Falls gewünscht, können die so erhaltenen Hydrochloride der Verbindungen der allgemeinen Formel I auf üblichem Wege in die freien Aminosäuren überführt werden.

Auch bei Verfahrensschritt b) tritt überraschenderweise keine Racemisierung ein, obwohl dies für die verwandten 1,3-Oxazolin-5-on-Verbindungen bekannt ist (Houben-Weyl, Band XV/1, S. 35 ff).

Zur Vermeidung unerwünschter Nebenreaktionen wird auch bei Verfahrensschritt b) vorteilhafterweise unter einer Schutzgasatmosphäre, beispielsweise unter Argon, Stickstoff oder Kohlendioxid gearbeitet.

Die alkalische Hydrolyse mittels Alkalihydroxiden wie NaOH, KOH oder Alkalialkoholaten erfolgt analog der in Chem. Pharm. Bull. 17, 1679 (1969) beschriebenen Verfahrensweisen.

Die Umsetzung der Verbindungen der Formel IV mit Ammoniak, Ala-Ala-$OR_6$ bzw. Ala-Leu-$OR_6$ erfolgt auf übliche Weise (Houben-Weyl XV/2, S. 91 ff).

Die erhaltenen Verbindungen der Formel I können nach bekannten Verfahren entweder in ihre Salze überführt werden oder die Reste $R_1$, $R_2$, $R_3$, $R_4$, $R_5$ oder $R_6$ auf geeignete Weise abgespalten werden.

Die Salzbildung erfolgt mittels dem Fachmann geläufigen Verfahren. Die Salze der Verbindungen I sind aus DE-OS 2 856 260 bekannt.

Die Abspaltungsreaktionen der Reste $R_1$, $R_2$, $R_3$, $R_4$, $R_5$ oder $R_6$ sind ebenfalls an sich bekannt.

Falls $R_3$ = $-COOCH_2C_6H_5$ und $R_6$ = $CH_2C_6H_5$ bedeutet, gelingt die Abspaltung beispielsweise durch katalytische Hydrierung. Die Abspaltung des Rests $R_1$ (für n = 1) oder $R_2$ (für m = 1) gelingt mittels Mineralsäuren oder speziellen Reagentien wie Natriumjodid/Trimethylchlorsilan in Acetonitril (Houben-Weyl E2, S. 312 ff und 322 ff) oder Bromtrimethylsilan (Tetrahedron Letters 1977, 155).

Die Zwischenverbindungen der Formel IV sind neu und sind daher ebenfalls Gegenstand der vorliegenden Erfindung.

Die nachfolgenden Beispiele dienen zur Erläuterung der Erfindung.

A. Herstellung der Zwischenprodukte (Verfahrensschritt a))

**Beispiel 1**

L-3-Benzyloxycarbonyl-4[2-[isoamoxy(methyl)phosphinyl]ethyl] 1,3-oxazolidin-5-on

5,0 g (0,033 mol) Methanphosphonigsäureisoamylester werden unter Argonatmosphäre auf 90 °C erhitzt und innerhalb von 10 min tropfenweise mit einer Lösung von 2,7 g (0,011 mol) L-3-Benzyloxycarbonyl-4-vinyl-1,3-oxazolidin-5-on und 0,024 g (0,00011 mol) tert.-Butylperneodecanoat in 4 ml Xylol versetzt. Nach beendeter Zugabe wird noch 60 min bei 90 °C weitergerührt, dann wird der überschüssige Methanphosphonigsäureisoamylester im Hochvakuum abdestilliert. Das Rohprodukt wird durch Chromatographie an Kieselgel gereinigt (Laufmittel: Dichlormethan/Methanol). Man erhält 0,8 g (18,3 % der Theorie) L-3-Benzyloxycarbonyl-4-[2-[isoamoxy(methyl)phosphinyl]ethyl]-1,3-oxazolidin-5-on als farbloses Öl.
$^{31}$P-NMR ($CDCl_3$): 52,710
$[\alpha]_D^{23}$ : 74,4 ° (c = 0,46 in $CHCl_3$)

**Beispiel 2**

L-3-Benzyloxycarbonyl-4[2-[isobutoxy(methyl)phosphinyl]ethyl]-1,3-oxazolidin-5-on

2,0 g (0,008 mol) L-3-Benzyloxycarbonyl-4-vinyl-1,3-oxazolidin-5-on und 3,2 g (0,024 mol) Methanphosphonigsäureisobutylester werden in 6 ml Xylol gelöst und unter Argonatmosphäre auf 125 °C erhitzt. In die heiße Reaktionsmischung werden 0,022 g (0,0001 mol) tert.Butylperethylhexanoat getropft. Nach 30 min bzw. 60 min werden nochmals je 0,022 g (0,0001 mol) tert.-Butylperethylhexanoat zugetropft.

Nach der letzten Zugabe wird noch 60 min bei 125 °C gerührt. Der überschüssige Methanphosphonigsäureisobutylester wird im Hochvakuum abdestilliert und das Rohprodukt an Kieselgel chromatographiert (Laufmittel: Dichlormethan/Methanol). Man erhält 2,20 g (71,1 % der Theorie) L-3-Benzyloxy-carbonyl-4-[2-[isobutoxy(methyl)phosphinyl]ethyl]-1,3-oxazolidin-5-on als farbloses Öl.
$^1$H-NMR ($CDCl_3$): 0,93 (d, J = 7Hz, 6H), 1,43 (d, J = 14Hz, 3H), 1,68-2,38 (m, 5H), 3,72 (t, J = 7Hz, 2H), 4,38 (t, J = 5,5Hz, 1H), 5,19 (s, 2H), 5,24 (d, J = 5Hz, 1H), 5,53 (d, J = 5Hz, 1H), 7,38 (s, 5H)
$^{31}$P-NTR ($CDCl_3$) : 52,852

$$[\alpha]_D^{19,5}:$$

72,6 ° (c = 0,67 in $CHCl_3$)

| | | | | |
|---|---|---|---|---|
| $C_{18}H_{26}NO_6P$ (383,373) | Ber.:<br>Gef.: | C 56,4<br>C 56,3 | H 6,8<br>H 6,8 | N 3,6<br>N 3,6 |

**Beispiel 3**

L-3-Benzyloxycarbonyl-4-[2-[ethoxy(methyl)phosphinyl]ethyl]-1,3-oxazolidin-5-on

2,0 g (0,0182 mol) Methanphosphonigsäureethylester werden unter Argonatmosphäre auf 120 °C erhitzt und innerhalb von 15 min tropfenweise mit einer Lösung von 1,5 g (0,006 mol) L-3-Benzyloxycarbonyl-4-vinyl-1,3-oxazolidin-5-on und 0,032 g (0,00015 mol) tert.-Butylperethylhexanoat in 4 ml Xylol versetzt. Nach beendeter Zugabe wird noch 70 min bei 120 °C weitergerührt, dann wird der überschüssige Methanphosphonigsäureethylester im Hochvakuum abdestilliert. Das Rohprodukt wird durch Chromatographie an Kieselgel gereinigt (Laufmittel: Dichlormethan/Methanol). Man erhält 2,1 g (97,4 % der Theorie) L-3-Benzyloxycarbonyl-4-[2-[ethoxy(methyl)phosphinyl]ethyl]-1,3 oxazolidin-5-on als farbloses Öl.
$^1$H-NMR ($CDCl_3$): 1,30 (t, J = 7Hz, 3H), 1,43 (d, J = 14Hz, 3H), 1,57-2,48 (m, 4H), 4,03 (quint, J = 7Hz, 2H), 4,37 (t, J = 5,5Hz, 1H), 5,19 (s, 2H), 5,24 (d, J = 5Hz, 1H), 5,53 (d, J = 5Hz, 1H) 7,40 (s, 5H)
$^{31}$P-NMR ($CDCl_3$) : 52,780
$[\alpha]_D^{23}$ : 79,7 ° (c = 0,49 in $CHCl_3$)

**Beispiel 4**

L-3-Benzyloxycarbonyl-4[2-[cyclohexyloxy(methyl)phosphinyl]ethyl]-1,3-oxazolidin-5-on

Unter Einsatz von Methanphosphonigsäurecyclohexylester erhält man analog zu Beispiel 3 89,0 % der Theorie L-3-Benzyloxycarbonyl-4[2-[cyclohexyloxy(methyl)phosphinyl]ethyl]-1,3-oxazolidin-5-on als farbloses Öl.

$^{1}$H-NMR ($CDCl_3$): 1,43 (d, J=14Hz, 3H) überlagert durch 1,10-2,45 (m, 14H), 4,37 (t, J=5,5Hz, 1H), 5,19 (s, 2H), 5,23 (d, J=5Hz, 1H), 5,52 (d, J=5Hz, 1H), 7,38 (s, 5H)
$^{31}$P-NMR ($CDCl_3$) : 51,364
$[\alpha]_D^{23}$ : 69,4° (c = 0,59 in $CHCl_3$)

**Beispiel 5**

L-3-Benzyloxycarbonyl-4[2-(dimethylphosphinyl)ethyl]-1,3-oxazolidin-5-on

2,6 g (0,033 mol) Dimethylphosphanoxid werden unter Argonatmosphäre auf 120°C erhitzt und innerhalb von 10 min tropfenweise mit einer Lösung 2,7 g (0,011 mol) L-3-Benzyloxycarbonyl-4-vinyl-1,3-oxazolidin-5-on und 0,040 g (0,00018 mol) tert.-Butylperethylhexanoat in 4,5 ml Xylol versetzt.

Nach beendeter Zugabe wird noch 55 min bei 120°C weitergerührt, dann wird das überschüssige Dimethylphosphanoxid im Hochvakuum abdestilliert. Das Rohprodukt wird durch Chromatographie an Kieselgel gereinigt (Laufmittel: Dichlormethan/Methanol). Man erhält 2,20 g (62,0 % der Theorie) L-3-Benzyloxycarbonyl-4[2-(dimethylphosphinyl)ethyl]-1,3-oxazolidin-5-on als farbloses Öl.

$^{1}$H-NMR ($CDCl_3$): 1,44 (d, J=14Hz, 6H) überlagert durch 1,30-2,50 (m, 4H), 4,35 (t, J=5,5Hz, 1H), 5,18 (s, 2H), 5,24 (d, J=5Hz, 1H), 5,50 (d, J=5Hz, 1H), 7,35 (s,5H)
$^{31}$P-NMR ($CDCl_3$) : 42,170
$[\alpha]_D^{23}$ : 81,7° (c = 0,4 in CHC13)

**Beispiel 6**

L-3-Benzyloxycarbonyl-4[2-diisopropoxyphosphinyl)ethyl]-1,3-oxazolidin-5-on

5,1 g (0,031 mol) Diisopropylphosphit werden unter Argonatmosphäre auf 120°C erhitzt und innerhalb von 25 min tropfenweise mit einer Lösung von 1,9 g (0,0077 mol) L-3-Benzyloxycarbonyl-4-vinyl-1,3-oxazolidin-5-on und 0,12 g (0,00055 mol) tert.-Butylperethylhexanoat in 4 ml Xylol versetzt. Die Mischung wird 60 min bei 120°C gerührt, dann werden nochmals 0,12 g (0,00055 mol) tert.-Butylperethylhexanoat zugegeben und weitere 60 min bei 120°C gerührt. Das überschüssige Diisopropylphosphit sowie das Lösungsmittel werden unter reduziertem Druck (Anlegen von Hochvakuum) abdestilliert. Das Rohprodukt wird durch Chromatographie an Kieselgel gereinigt (Laufmittel: Dichlormethan/Methanol).

Man erhält 1,95 g (61,4 % der Theorie) L-3-Benzyloxycarbonyl-4[2-(diisopropoxyphosphinyl)ethyl]-1,3-oxazolidin-5-on als farbloses Öl.

$^{1}$H-NMR ($CDCl_3$): 1,30 (d, J=6Hz, 12H), 1,47-2,45 (m, 4H), 4,36 (t, J=5,5Hz, 1H), 5,66 (m, 2H), 5,18 (s, 2H), 5,23 (d, J=5Hz, 1H), 5,53 (d, J=5Hz, 1H), 7,37 (s, 5H)
$^{31}$P-NMR ($CDCl_3$): 27,645
$[\alpha]_D^{21}$ : 56,6° (c = 0,59 in $CHCl_3$)

Ausgehend von L-2-Methyl-3-benzyloxycarbonyl-4-vinyl-1,3-oxazolidin-5-on können folgende Verbindungen der Formel IV erhalten werden:

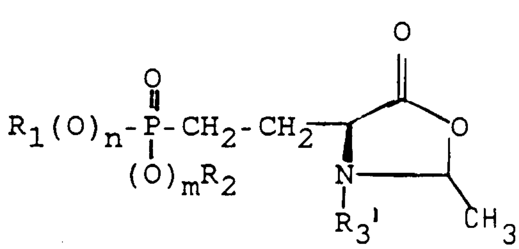

(IV)

| Beispiel | $R_1$ | $R_2$ | $R_3'$ | n | m | Ausbeute | $^{31}$P-NMR [δ] | $[\alpha]_D^{21}$ |
|---|---|---|---|---|---|---|---|---|
| 7 | $CH_3$ | $C_2H_5$ | $COOCH_2C_6H_5$ | 0 | 1 | 77,4% | 52,645<br>53,008 | 58,8° (c= 0,41, $CHCl_3$) |
| 8 | $CH(CH_3)_2$ | $CH(CH_3)_2$ | $COOCH_2C_6H_5$ | 1 | 1 | 65,5% | 27,747<br>27,900 | 31,6° (c= 0,45, $CHCl_3$) |
| 9 | $CH_3$ | $CH_3$ | $COOCH_2C_6H_5$ | 0 | 0 | 34,7% | | |

B. Herstellung der Endprodukte (Verfahrensschritt b))

**Beispiel 10**

L-Homoalanin-4-yl(methyl)phosphinsäurehydrochlorid

1,84 g (0,0048 mol) L-3-Benzyloxycarbonyl-4-[2-[isobutoxy(methyl)phosphinyl]ethyl]-1,3-oxazolidin-5-on werden in 60 ml 6N Salzsäure aufgenommen und unter Stickstoffatmosphäre 6 Stunden am Rückfluß gekocht. Die wäßrige Reaktionsmischung wird dreimal mit je 20 ml Dichlormethan extrahiert, mit wenig Aktivkohle versetzt, kurz aufgekocht und nach Abfiltrieren der Aktivkohle bis zur Trockne eingeengt. Die vereinigten Dichlormethan-Extrakte werden verworfen. Auf diese Weise erhält man 0,95 g (91,0 % der Theorie) L-Homoalanin-4-yl(methyl)phosphinsäurehydrochlorid als leicht gelbliches Pulver, das durch sein $^1$H-NMR-Spektrum identifiziert wird.
$^1$H-NMR ($D_2O$): 1,55 (d, J = 14Hz, 3H), 1,71-2,43 (m, 4H), 4,16 (t, J = 5,5Hz, 1H)
Der Enantiomerenüberschuß, der mit Hilfe einer HPLC-Methode [J. Chromatogr. 368, 413 (1986)] bestimmt wurde, beträgt 97,4 % ee.

**Beispiel 11**

L-Homoalanin-4-yl(methyl)phosphinsäure

Ausgehend von L-2-Methyl-3-benzyloxycarbonyl-4-[2-[ethoxy(methyl)phosphinyl]ethyl]-1,3-oxazolidin-5-on (Beispiel 7) erhält man analog zu Beispiel 10 91,1 % der Theorie L-Homoalanin-4-yl(methyl)-phosphinsäurehydrochlorid mit einem Enantiomerenüberschuß von 97,0 % ee, das in üblicher Weise durch Umsetzung mit ca. der doppelt molaren Menge Propenoxid in die freie Aminosäure überführt wird.
$^1$H-NMR ($D_2O$): 1,38 (d, J = 14Hz, 3H), 1,50-2,35 (m, 4H), 3,97 (t, J = 5,5Hz, 1H)
Fp = 208-211 °C (Zers.)
$[\alpha]_D^{19}$ = 16,1 ° (c = 0,70 in $H_2O$)
Dies entspricht einer optischen Ausbeute von mindestens 94,7 %, bezogen auf $[\alpha]_D^{23}$ = 17,0 ° (c = 1,00 in $H_2O$) für optisch reine L-Homoalanin-4-yl(methyl)phosphinsäure (Sci. Reports of Meiji Seika Kaisha 13, 42 (1973)).

**Beispiel 12**

L-2-Amino-4-phosphonobuttersäure

1,15 g (0,0028 mol) L-3-Benzyloxycarbonyl-4-[2-(diisopropoxyphosphinyl)ethyl]-1,3-oxazolidin-5-on (Beispiel 6) werden in 25 ml 6N Salzsäure gelöst und unter Stickstoffatmosphäre 10 Stunden am Rückfluß gekocht. Die wässrige Reaktionsmischung wird dreimal mit je 15 ml Dichlormethan extrahiert, mit wenig Aktivkohle versetzt, kurz aufgekocht und nach dem Abfiltrieren der Aktivkohle zur Trockne eingeengt. Die vereinigten organischen Extrakte werden verworfen. Man erhält 0,52 g (84,6 %) L-2-Amino-4-phosphonobuttersäurehydrochlorid als glasartig erstarrenden Feststoff, der in 15 ml Ethanol/Wasser aufgenommen wird.

Durch Zugabe von 0,5 ml [≙ 0,42 g (0,0072 mol)] Propenoxid erhält man 0,24 g (46,8 % der Theorie) L-2-Amino-4-phosphonobuttersäure.
$^1$H-NMR ($D_2O$): 1,50-2,37 (m, 4H), 4,00 (t, J = 5,5Hz, 1H)
$[\alpha]_D^{20}$ : 16,7° (c = 0,55 in 6N HCl)

**Beispiel 13**

L-4-Dimethylphosphinyl-2-aminobuttersäurehydrochlorid

Analog zu Beispiel 12 erhält man ausgehend von L-3-Benzyloxycarbonyl-4[2-(dimethylphosphinyl)ethyl]-1,3-oxazolidin-5-on (Beispiel 5) 95,7 % der Theorie L-4-Dimethylphosphinyl-2-aminobuttersäurehydrochlorid als glasartig erstarrenden Feststoff.
$[\alpha]_D^{20}$ : 15,0° (c = 0,65 in 1N HCl)

**Beispiel 14**

L-(N-Benzyloxycarbonyl)-homoalanin-4-yl(methyl)phosphinsäureisobutylester

2,10 g (0,0055 mol) L-3-Benzyloxycarbonyl-4-[2-[isobutoxy(methyl)phosphinyl]ethyl]-1,3-oxazolidin-5-on (Beispiel 2) werden in 11 ml Tetrahydrofuran gelöst und mit 11 ml 1N Natronlauge versetzt. Die Mischung wird 1 Stunde bei Raumtemperatur gerührt und anschließend im Wasserstrahlvakuum eingeengt. Der Rückstand wird durch Zugabe von 11 ml 1N Salzsäure sauergestellt und die wässrige Lösung dreimal mit je 20 ml Dichlormethan extrahiert. Die vereinigten, organischen Extrakte werden über Natriumsulfat getrocknet und zur Trockne eingeengt. Man erhält 1,62 g (79,3 % der Theorie) L-(N-Benzyloxycarbonyl)-homoalanin-4-yl(methyl)phosphinsäureisobutylester als glasartig erstarrenden Feststoff.
$^{31}$P-NMR ($CDCl_3$): 58.550, 58.652

$$[\alpha]_D^{19,5} :$$

: 32,4° (c = 1,06 in $CHCl_3$)

| | | | | |
|---|---|---|---|---|
| $C_{17}H_{26}NO_6P$ (371,363) | Ber.:<br>Gef.: | C 55,0<br>C 54,6 | H 7,1<br>H 6,8 | N 3,8<br>N 3,9 |

**Beispiel 15**

L-Homoalanin-4-yl(methyl)phosphinsäureisobutylester

1,00 g (0,0027 mol) L-(N-Benzyloxycarbonyl)-homoalanin-4-yl(methyl)phosphinsäureisobutylester (Beispiel 14) werden in 25 ml Methanol gelöst, mit 0,1 g Palladium auf Aktivkohle (10 %) versetzt und 24 h bei Raumtemperatur und einem Druck von 3,0 bar hydriert. Nach dem Entspannen des Reaktionsgefäßes wird der Katalysator abfiltriert und das Filtrat zur Trockne eingeengt. Durch Aufnehmen des Rückstand in Aceton/Wasser erhält man 0,54 g (84,3 % der Theorie) L-Homoalanin-4-yl(methyl)-phosphinsäureisobutylester. Fp.: 183-84°C (Zers.)
$^{31}$P-NMR ($D_2O$) : 61,466
$[\alpha]_D^{21}$ : 11,8° (c = 1,06 in $H_2O$)

| | | | | |
|---|---|---|---|---|
| $C_9H_{20}NO_4P$ (237,233) | Ber.:<br>Gef.: | C 45,6<br>C 46,2 | H 8,5<br>H 8,2 | N 5,9<br>N 5,9 |

**Beispiel 16**

L-4-[Isobutoxy(methyl)phosphinyl]-2-[(benzyloxycarbonyl)(hydroxymethyl)amino]butyryl-L-alanyl-L-alaninmethylester

0,53 g (0,0025 mol) L-Alanyl-L-alaninmethylesterhydrochlorid werden in 5 ml Tetrahydrofuran suspendiert und bei -20 °C mit 0,23 g (0,0022 mol) Triethylamin versetzt. Nach 20 min Rühren bei -20 °C wird eine Lösung von 0,86 g (0,0022 mol) L-3-Benzyloxycarbonyl-4-[2-[isobutoxy(methyl)phosphinyl]ethyl]-1,3-oxazolidin-5-on in 5 ml Tetrahydrofuran zugetropft. Die Mischung wird 30 min bei -20 °C gerührt, dann wird auf Raumtemperatur erwärmt und anschließend bei 50 °C gerührt. Der ausgefallene Niederschlag wird abfiltriert und das Filtrat im Hochvakuum vollständig eingeengt. Man erhält 1,10 g eines zähen Öls, das heiß in Toluol aufgenommen und filtriert wird. Nach dem Einengen des Filtrats im Hochvakuum verbleiben 0,85 g (71,6 % der Theorie) L-4-[Isobutoxy(methyl)phosphinyl]-2-[(benzyloxycarbonyl)(hydroxymethyl)amino]-butyryl-L-alanyl-L-alaninmethylester als farbloses Öl.
$^1$H-NMR ($d_6$-DMSO): 0,86 (d, J = 7Hz, 6H), 1,28 (d, J = 7Hz, 3H), 1,32 (d, J = 7Hz, 3H), 1,36 (d, J = 14Hz, 3H), 1,10-2,22 (m, 5H), 3,62 (s, 3H), 3,63 (t, J = 7Hz, 2H), 4,27 (m, 3H), 4,84 (m, breit, 2H), 5,10 (s, 2H), 7,35 (s, 5H), 8,10 (s, breit, 1H), 8,35 (d, breit 7Hz, 1H).

## Patentansprüche

**1.** Verfahren zur Herstellung von phosphorhaltigen L-Aminosäuren der Formel I oder deren Salze,

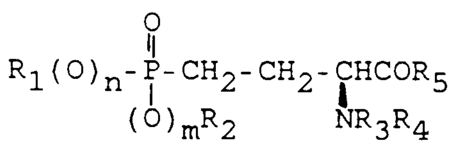

(I),

worin

| | |
|---|---|
| $R_1$ und $R_2$ | unabhängig voneinander Wasserstoff, ($C_1$-$C_6$)-Alkyl, das unsubstituiert ist oder durch Halogen oder ($C_6$-$C_{10}$)-Aryl ein- oder mehrfach substituiert ist, oder ($C_3$-$C_{10}$)-Cycloalkyl, |
| $R_3$ | Wasserstoff, ($C_1$-$C_6$)-Alkyl, das unsubstituiert ist oder ein- oder mehrfach durch Halogen und/oder einfach durch Hydroxy substituiert ist, ($C_1$-$C_{10}$)-Acyl,das ein- oder mehrfach durch Halogen oder ($C_1$-$C_6$)-Alkyl oder einfach durch ($C_6$-$C_{10}$)-Aryl, ($C_1$-$C_6$)-Alkoxy oder ($C_1$-$C_6$)-Alkoxy, das durch ($C_6$-$C_{13}$)-Aryl substituiert ist, substituiert sein kann, |
| $R_4$ | Wasserstoff oder 1-Hydroxy-($C_1$-$C_6$)-alkyl, das unsubstituiert oder ein- oder mehrfach durch Halogen substituiert ist, |
| $R_5$ | Hydroxy, ($C_1$-$C_6$)-Alkoxy, Amino, Ala-Ala-$OR_6$ oder Ala-Leu-$OR_6$, |
| $R_6$ | Wasserstoff, ($C_1$-$C_6$)-Alkyl oder Benzyl, |
| n | die Zahl null oder eins und |
| m | die Zahl null, wenn $R_2$ nicht Wasserstoff ist, oder 1 |

bedeuten, dadurch gekennzeichnet, daß man

a) eine Verbindung der Formel II

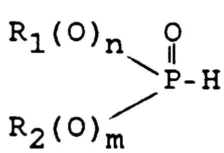

(II),

wobei $R_1$, $R_2$ die obengenannte Bedeutung besitzt mit der Ausnahme, daß $R_1$ nicht Wasserstoff sein darf, wenn n = 1 ist, mit einer Verbindung der Formel III

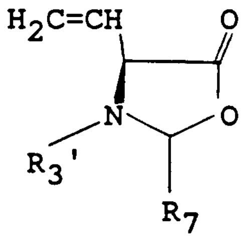

(III),

worin

$R_3'$ die Bedeutung von $R_3$ außer Wasserstoff besitzt und

$R_7$ H, ($C_1$-$C_5$)-Alkyl, das ein- oder mehrfach durch Halogen substituiert sein kann, bedeutet, in Gegenwart katalytischer Mengen eines Radikalbildners umsetzt und

b) das erhaltene Zwischenprodukt der Formel IV

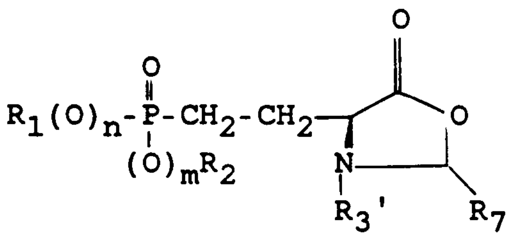

(IV)

mit HCl, einem Alkalihydroxid, Alkalialkoholat, $NH_3$, H-Ala-Ala-$OR_6$ oder H-Ala-Leu-$OR_6$, wobei $R_6$ die obengenannte Bedeutung außer Wasserstoff besitzt, umsetzt und die erhaltene Verbindung der Formel I gegebenenfalls auf üblichem Wege derivatisiert.

**2.** Verfahren gemäß Anspruch 1, dadurch gekennzeichnet, daß in Formel I $R_1(O)_n$ = $CH_3$ oder $C_2H_5$, $R_2$-$(O)_m$ = OH, $R_3$ und $R_4$ = H und $R_5$ = OH, Ala-Ala-OH oder Ala-Leu-OH bedeuten.

**3.** Verfahren gemäß Ansprüchen 1 oder 2, dadurch gekennzeichnet, daß der Verfahrensschritt a) im Temperaturbereich zwischen 50° und 200°C durchgeführt wird.

**4.** Verfahren gemäß Anspruch 1 oder 2, dadurch gekennzeichnet, daß der Verfahrensschritt a) im Temperaturbereich zwischen 70° und 140°C durchgeführt wird.

**5.** Verfahren gemäß einem oder mehreren der Ansprüche 1 bis 4, dadurch gekennzeichnet, daß man im Verfahrensschritt a) als Radikalbildner organische Peroxide, Carbonsäureperester oder aliphatische Azoverbindungen verwendet.

**6.** Verfahren nach Anspruch 5, dadurch gekennzeichnet, daß man als Radikalbildner Carbonsäureperester verwendet.

**7.** Verfahren nach Anspruch 5 oder 6, dadurch gekennzeichnet, daß man den Radikalbildner in einer Menge von 0,1 bis 20 Molprozent, bezogen auf die Komponente III, einsetzt.

**8.** Verfahren gemäß einem oder mehreren der Ansprüche 1 bis 7, dadurch gekennzeichnet, daß man beim Verfahrensschritt b) wäßrige 3N bis 12N HCl verwendet.

**9.** Verfahren gemäß Anspruch 8, dadurch gekennzeichnet, daß man die Umsetzung mit HCl bei 90° bis 130°C durchführt.

**10.** Verbindungen der in Anspruch 1 definierten Formel IV.

## Claims

**1.** A process for the preparation of phosphorus-containing L-amino acids of the formula I or their salts

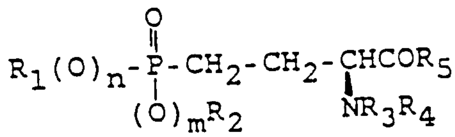

(I)

in which

- $R_1$ and $R_2$ independently of one another denote hydrogen, $(C_1\text{-}C_6)$-alkyl which is unsubstituted, mono-substituted or polysubstituted by halogen or $(C_6\text{-}C_{10})$-aryl, or $(C_3\text{-}C_{10})$-cycloalkyl,
- $R_3$ denotes hydrogen, $(C_1\text{-}C_6)$-alkyl which is unsubstituted, monosubstituted or polysubstituted by halogen and/or monosubstituted by hydroryl, $(C_1\text{-}C_{10})$-acyl which may be monosubstituted or polysubstituted by halogen or $(C_1\text{-}C_6)$-alkyl or monosubstituted by $(C_6\text{-}C_{10})$-aryl, $(C_1\text{-}C_6)$-alkoxy or $(C_1\text{-}C_6)$-alkoxy which is substituted by $(C_6\text{-}C_{13})$-aryl,
- $R_4$ denotes hydrogen or 1-hydrory-$(C_1\text{-}C_6)$-alkyl which is unsubstituted, monosubstituted or polysubstituted by halogen,
- $R_5$ denotes hydroxyl, $(C_1\text{-}C_6)$-alkoxy, amino, Ala-AlaOR$_6$ or Ala-Leu-OR$_6$,
- $R_6$ denotes hydrogen, $(C_1\text{-}C_6)$-alkyl or benzyl,
- n denotes the number zero or one and
- m denotes the number zero if $R_2$ is not hydrogen, or 1, which comprises reacting

a) a compound of the formula II

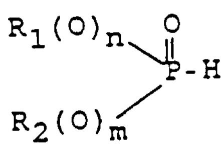

(II)

where $R_1$ and $R_2$ have the abovementioned meaning with the exception that $R_1$ is not hydrogen if n is 1, with a compound of the formula III

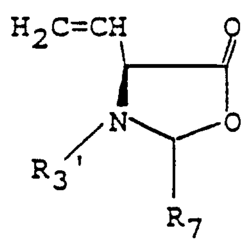

(III)

in which

- $R_3$' has the meaning of $R_3$ apart from hydrogen and
- $R_7$ denotes H, $(C_1\text{-}C_5)$-alkyl which may be mono-substituted or polysubstituted by halogen, in the presence of catalytic amounts of a radical former and

b) reacting the intermediate obtained of the formula IV

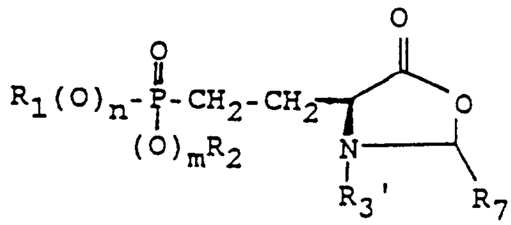

(IV)

with HCl, an alkali metal hydroxide, an alkali metal alkoxide, $NH_3$, H-Ala-Ala-$OR_6$ or H-Ala-Leu-$OR_6$, where $R_6$ has the abovementioned meaning apart from hydrogen, and optionally derivatizing the compound of the formula I obtained in a customary manner.

**2.** The process as claimed in claim 1, wherein, in formula I, $R_1(O)_n$ denotes $CH_3$ or $C_2H_5$, $R_2(O)_m$ denotes OH, $R_3$ and $R_4$ are H and $R_5$ denotes OH, Ala-Ala-OH or Ala-Leu-OH.

**3.** The process as claimed in in claims 1 or 2, wherein process step a) is carried out in a temperature range between 50° and 200°C.

**4.** The process as claimed in claim 1 or 2 wherein process step a) is carried out in a temperature range between 70° and 140°C.

**5.** The process as claimed in one or more of claims 1 to 4, wherein organic peroxides, carboxylic acid peresters or aliphatic azo compounds are used as radical formers in process step a).

**6.** The process as claimed in claim 5, wherein carboxylic acid peresters are used as radical formers.

**7.** The process as claimed in claim 5 or 6, wherein the radical former is employed in an amount from 0.1 to 20 mol percent, relative to the component III.

**8.** The process as claimed in one or more of claims 1 to 7, wherein aqueous 3N to 12N HCl is used in process step b).

**9.** The process as claimed in claim 8, wherein the reaction with HCl is carried out at 90° to 130°C.

**10.** A compound of the formula IV defined in claim 1.

## Revendications

**1.** Procédé pour préparer des acides L-aminés phosphorés de formule I, ou leurs sels

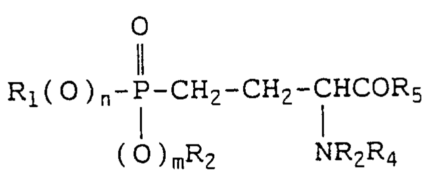

(I)

où

$R_1$ et $R_2$, indépendamment l'un de l'autre, sont chacun un hydrogène, un radical alkyle en $C_1$-$C_6$ non substitué, ou une ou plusieurs fois substitué par des radicaux halogéno ou aryle en $C_6$-$C_{10}$, ou encore cycloalkyle en $C_3$-$C_{10}$,

$R_3$ est un hydrogène, un radical alkyle en $C_1$-$C_6$ qui est non substitué ou est une ou plusieurs fois substitué par des radicaux halogéno et/ou une fois par un radical hydroxy, acyle en $C_1$-$C_{10}$ pouvant être une ou plusieurs fois substitué par des radicaux halogéno ou alkyle en $C_1$-$C_6$ ou une fois substitué par un radical aryle en $C_6$-$C_{10}$, alcoxy en $C_1$-$C_6$ ou alcoxy en $C_1$-$C_6$ substitué par des radicaux aryle

en $C_6$-$C_{13}$,
$R_4$ est un hydrogène ou un radical hydroxy-1 alkyle en $C_1$-$C_6$, qui est non substitué ou une ou plusieurs fois substitué par des radicaux halogéno,
$R_5$ est un radical hydroxy, alcoxy en $C_1$-$C_6$, amino, Ala-Ala-$OR_6$ ou Ala-Leu-$OR_6$,
$R_6$ est un hydrogène ou un radical alkyle en $C_1$-$C_6$ ou benzyle,
n est le nombre 0 ou 1, et
m est le nombre 0 quand $R_2$ n'est pas un hydrogène, ou 1,
caractérisé en ce que

a) on fait réagir en présence de quantités catalytiques d'un agent formant des radicaux, un composé de formule II

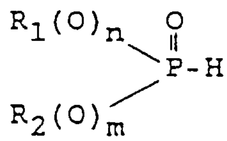

(II)

dans laquelle $R_1$ et $R_2$ ont les significations données ci-dessus, sauf que $R_1$ ne peut être un hydrogène quand n = 1,
avec un composé de formule III

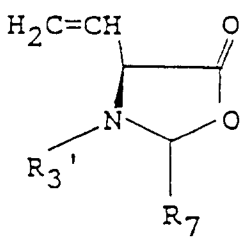

(III)

dans laquelle $R_3$' a les significations de $R_3$ à l'exception de l'hydrogène, et $R_7$ est H ou un radical alkyle en $C_1$-$C_5$ qui est non substitué ou est une ou plusieurs fois substitué par des radicaux halogéno, et

b) on fait réagir le produit intermédiaire obtenu de formule IV

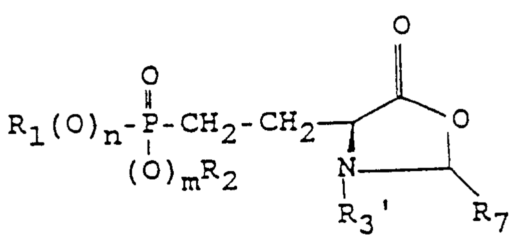

(IV)

avec HCl, un hydroxyde d'un métal alcalin, un alcoolate d'un métal alcalin, $NH_3$, H-Ala-Ala-$OR_6$ ou H-Ala-Leu-$OR_6$, où $R_6$ a les significations données ci-dessus à l'exception de l'hydrogène,

le composé obtenu de formule I étant ensuite éventuellement traité de la manière habituelle pour donner un dérivé.

**2.** Procédé selon la revendication 1, caractérisé en ce que, dans la formule I, $R_1(O)_n$ = $CH_3$ ou $C_2H_5$, $R_2$-$(O)_m$ = OH, $R_3$ et $R_4$ = H, et $R_5$ = OH, Ala-Ala-OH ou Ala-Leu-OH.

**3.** Procédé selon la revendication 1 ou 2, caractérisé en ce que l'étape a) est mise en oeuvre à une température comprise entre 50 et 200 °C.

**4.** Procédé selon la revendication 1 ou 2, caractérisé en ce que l'étape a) est mise en oeuvre à une température comprise entre 70 et 140 °C.

**5.** Procédé selon l'une ou plusieurs des revendications 1 à 4, caractérisé en ce qu'on utilise dans l'étape a) comme agent formant des radicaux des peroxydes organiques, des percarboxylates ou des composés azoïques aliphatiques.

**6.** Procédé selon la revendication 5, caractérisé en ce qu'on utilise comme agents formant des radicaux des percarboxylates.

**7.** Procédé selon la revendication 5 ou 6, caractérisé en ce qu'on utilise l'agent formant des radicaux en une quantité de 0,1 à 20 % en moles par rapport au composant III.

**8.** Procédé selon l'une ou plusieurs des revendications 1 à 7, caractérisé en ce qu'on utilise dans l'étape b) du HCl en solution aqueuse 3 N à 12 N.

**9.** Procédé selon la revendication 8, caractérisé en ce que la réaction avec HCl est mise en oeuvre à une température de 90 à 130 °C.

**10.** Composés ayant la formule IV définie dans la revendication 1.